Europäisches Patentamt

European Patent Office

Office européen des brevets

(19)

(11) Veröffentlichungsnummer: **0 338 395 B1**

# (12) EUROPÄISCHE PATENTSCHRIFT

(45) Veröffentlichungstag der Patentschrift: **28.12.94**

(21) Anmeldenummer: **89106463.6**

(22) Anmeldetag: **12.04.89**

(51) Int. Cl.5: **C12P 21/08**, C12N 15/13, C12N 5/12, A61K 39/104, G01N 33/577

(54) **Monoklonaler Antikörper gegen Pseudomonas aeruginosa, seine Herstellung und Verwendung.**

(30) Priorität: **19.04.88 DE 3813023**

(43) Veröffentlichungstag der Anmeldung:
**25.10.89 Patentblatt 89/43**

(45) Bekanntmachung des Hinweises auf die Patenterteilung:
**28.12.94 Patentblatt 94/52**

(84) Benannte Vertragsstaaten:
**AT BE CH DE ES FR GB GR IT LI LU NL SE**

(56) Entgegenhaltungen:
**EP-A- 0 256 713**
**EP-A- 0 270 077**

**GENE, Band 74, Dezember 1988, Seiten 335-345, Elsevier Science Publishers B.V.(Biomedical Division); M. MARGET et al.: "Cloning and characterization of cDNAscoding for the heavy and light chains of a monoclonal antibody specific for Pseudomonas aeruginosa outer membrane protein I"**

**CHEMICAL ABSTRACTS, Band 110, Nr. 13, 27.**

**März 1989, Zusammenfassung Nr.109427k, Columbus, Ohio, US;**

**INFECTION AND IMMUNITY, Band 42, Nr. 3, Dezember 1983, Seiten 1027-1033,American Society for Microbiology; L.M. MUTHARIA et al.: "Surface localizationof Pseudomonas aeruginosa outer membrane porin protein F by using monoclonalantibodies"**

(73) Patentinhaber: **BEHRINGWERKE Aktiengesellschaft**
**Postfach 1140**
**D-35001 Marburg (DE)**

(72) Erfinder: **Domdey, Horst, Dr.**
**Fasanenweg 6**
**D-8027 Neuried (DE)**
Erfinder: **Marget, Matthias**
**Olgastrasse 12**
**D-8000 München 19 (DE)**
Erfinder: **von Specht, Bernd-Ulrich, Prof. Dr.**
**Am Waldweg**
**D-8193 Ambach (DE)**

## Beschreibung

Die Erfindung betrifft einen monoklonalen Antikörper (mAK), der mit allen bisher bekannten 19 Pseudomonas aeruginosa-Serotypen kreuzreagiert. Dieser Antikörper kann als Diagnostikum, als Wirkstoff oder als Wirkstoffträger eingesetzt werden.

Pseudomonas aeruginosa ist ein opportunistisches, gramnegatives, pathogenes Bakterium. Es kann lebensbedrohliche Krankheiten verursachen, z. B. Pneumonie bei Patienten mit zystischer Fibrose oder Septikämie bei Patienten mit Brandverletzungen bzw. Knochen- oder Harnwegsinfektionen. Die Therapie von durch Pseudomonas aeruginosa verursachten Erkrankungen mittels Antibiotika ist durch sein Resistenzmuster schwierig und oft erfolglos. Deshalb richtete sich die Aufmerksamkeit bei der Infektionsbekämpfung auf Immunprophylaxe und Immuntherapie. Im Rahmen von Studien zur Antigenität von Pseudomonas aeruginosa wurden monoklonale Antikörper gegen das äußere Membranprotein F hergestellt. Diese mAK reagierten mit den 17 untersuchten Serotypen von Pseudomonas aeruginosa sowie mit Isolaten von Patienten mit zystischer Fibrose (L. M. Mutharia et al. (1983) Infection and Immunity, Vol. 42, No. 3, 1027-1033). Gemäß späteren Untersuchungen ergab Schutz gegen alle 19 Serotypen von Pseudomonas aeruginosa eine Mischung von gereinigten äußeren Membranproteinen (OMP) F, H2 und I (von Specht et al. (1987) Infection 15, 408-412). Bei dieser Untersuchung wurden aus immunisierten Mäusen Lymphozyten isoliert und in bekannter Weise mit NS-1 Myelomzellen (Kearny et al. (1979), J. Immunology, 123, 4, 1548-1550) fusioniert. Aus etwa 500 erhaltenen Hybridomen wurde ein mAK - im folgenden mAK 6A4 bezeichnet - ausgewählt, der die nachfolgend aufgezählten vorteilhaften Eigenschaften besitzt:
- starke und einheitliche Reaktion mit allen 19 Pseudomonas aeruginosa Serotypen
- positiver C1q Bindungstest
- schützend bei Belastungsinfektion von Mäusen mit Pseudomonas aeruginosa.

Zusätzlich wurde die cDNA bestimmt (siehe Beispiele, Tab. 1 und Tab. 2). Ein humaner mAK kann nun erhalten werden, indem die konstanten Genbereiche des Mausantikörpers durch die entsprechenden humanen Sequenzen ersetzt werden. Die Expression dieser "humanisierten" Antikörpersequenz in eukaryotischen Zellen, zum Beispiel CHO-Zellen oder Hefen, liefert den entsprechenden humanen mAK, der für diagnostische Zwecke, für Prophylaxe und Therapie eingesetzt werden kann.

Die Erfindung betrifft folglich

a) gereinigte und isolierte DNA-Sequenzen, die für mAK 6A4 kodieren oder Teile davon, einschließlich ihrer Transkriptionsprodukte, sowie die entsprechende Kombination der "humanisierten" Sequenzen,

b) diese Sequenzen ganz oder teilweise enthaltende DNA-Strukturen und Vektoren,

c) mit solcher DNA transformierte pro- oder eukaryotische Zellen,

d) die von diesen Zellen aufgrund der Transformation exprimierten Polypeptide oder Teile davon,

e) deren Aminosäuresequenzen,

f) Diagnostika, Prophylaktika oder Therapeutika, die Peptide oder Aminosäuresequenzen von (e) alleine oder in Kombination enthalten,

g) ein Verfahren zur gentechnischen Herstellung der unter (d) genannten Polypeptide oder Teilen davon,

h) sowie das Epitop, an das Antikörper kodiert von Sequenzen unter (a) binden.

Weitere Ausgestaltungen der Erfindung sind in den nachfolgenden Beispielen, Tabellen und den Patentansprüchen aufgeführt.

Beispiel 1: Herstellung des mAK 6A4

1a) Reinigung der zur Immunisierung zu verwendenden äußeren Membranproteine OMP F, H2 und I von Pseudomonas aeruginosa Serotyp 12

OMP F, H2 und I wurden wie von T. Mizuno und M. Kageyama in J. Biochem. 84, 179-258 (1978) beschrieben aus Pseudomonas aeruginosa Serotyp 12 gereinigt. Demgemäß wurden die Zellen in der späten Log-Phase geerntet, durch Ultraschallbehandlung aufgebrochen und die Zellmembranen durch einstündige Zentrifugation bei 100 000 g gesammelt. Die Zellmembranen wurden schrittweise mit 2% SDS, 10% Glycerin und 2% SDS und schließlich 0.1 M NaCl extrahiert. Die übriggebliebene unlösliche Fraktion enthält hauptsächlich OMP F, OMP H2 und etwas OMP I.

1b) Immunisierung von Balb/c Mäusen mit OMP F, H2 und I

Zur Immunisierung erhielt jede Maus 50 $\mu$g gereinigte OMP's (80 $\mu$l) in einer Suspension mit 20 $\mu$g Al-(OH)$_3$ i.p. an Tag 1, was mit derselben Dosis an Tag 14 und Tag 21 wiederholt wurde. Nach weiteren 4 Wochen wurde nochmals geboostert (gleiche Dosis) und nach anschließend 3 Tagen die Milzen entnommen.

1c) Zellfusion

Bei der Zellfusion wurde im wesentlichen nach der Methode von G. Köhler und C. Milstein, Eur. J. Immunol. 6, 511 - 519 (1976) vorgegangen. Etwa $1\times10^8$ Milzzellen präpariert aus den entnommenen Milzen von (1b) wurden mit $5 \times 10^7$ NS-1 Myelomzellen (Kearny et al., a.a.O., kommerziell erhältlich von der American Type Culture Collection über ATCC Nr. TIB 18) in 2ml 50% Polyethylenglycol 1500 (Serva, Heidelberg) fusioniert. Nach der Fusion wurden die Zellen mit $1 \times 10^6$/ml normalen Milzzellen in Dulbeccos modifiziertem Minimalmedium (MEM) ausplattiert, das Hypoxanthin, Aminopterin und Thymidin (HAT) enthält. Antikörper-Produktion wurde durch den nachfolgend beschriebenen Enzyme-linked-Immuno-sorbent Assay (ELISA) geprüft und Klone von Interesse wurden durch Ausverdünnen gewonnen.

1d) ELISA zum Nachweis von Antikörper-sezernierenden Klonen

Mit Hilfe eines direkten ELISA's wurde bestimmt, ob und in welchem Umfang die verschiedenen mAK an das Antigen binden.

Mikrotiterplatten der Firma Dynatech, Plochingen, aus Polyvinylchlorid, mit 96 Vertiefungen, wurden mit 100µl Pseudomonas aeruginosa Sonikat (s.u., 1 : 200 verdünnt mit PBS = 4,5µg/ml) des Serotyps 12 beschichtet und über Nacht bei 4°C inkubiert. Danach wurden die Platten dreimal gewaschen und 1 Stunde bei 37°C mit in PBS verdünntem bovinem Serumalbumin abgesättigt. Nach erneutem Waschen wurden die Vertiefungen mit Folie abgedeckt und die Platten bis zur Verwendung bei minus 20°C aufbewahrt.

Für jeden zu testenden Klon wurden (von einer logarithmischen Verdünnungsreihe 1 : 2, 1 : 4, 1 : 8, 1 : 16,...) jeweils 50µl Ascites (als Doppelproben) einpipettiert.

Als positive Kontrolle diente polyklonales Mausserum von mit den Membranproteinen aktiv immunisierten und 4-fach geboosterten Mäusen (1 : 100, 1 : 1000, 1 : 10 000). Als negatives Kontrollserum wurde das Serum nicht immunisierter Mäuse verwendet.

Als Verdünnungsmedium diente PBS (phosphate buffered saline). Nach einstündiger Inkubation der Platte bei 37°C und dreimaligem Waschen wurden 50µl Peroxydase konjugiertes Anti-Maus-F(ab)$_2$ in der Verdünnung 1 : 500 zugegeben.

Nach erneuter einstündiger Inkubation bei 37°C und dreimaligem Waschen wurden 50µl des Substrats o-Phenylendiamin (0,2% OPD, 0,015 % $H_2O_2$) zugegeben. Alle ELISA-Reagenzien stammten aus dem NEI-501 Kit der Fa. NEN New England Nuclear, Dreieich.

Nach weiterem dreißigminütigem Inkubieren wurde die Reaktion mit 50µl 4,5M $H_2SO_4$ gestoppt und die Extinktion bei 450nm mit einem ELISA-Auswertgerät MR 580 der Firma Dynatech bestimmt. Positive Klone zeigten eine Extinktion $\geq$ 0,2 OD.

Aus 7 Klonen der engeren Wahl wurde der Klon, der mAK 6A4 sekretiert, für die anschließenden Untersuchungen ausgewählt, weil mAK 6A4 die auf S. 2 geschilderten Vorteile besitzt und zusätzlich stark sekretiert wird (etwa 1% der gamma-Globulin-Fraktion in Ascites-Flüssigkeit besteht aus mAK 6A4). mAK 6A4 gehört zur IgG2a Klasse und reagiert mit OMP I.

Sonikatherstellung:

Die Bakterien des Serotyps 0-12 wurden in Trypticase-Soy-Boullion angezüchtet. Dann wurden sie 3x gewaschen und in Natrium-Chlorid-Puffer resuspendiert. Die Suspension wurde 3 x 5 Minuten (mit je 2 Minuten Abstand) beschallt.

Nach erneutem Zentrifugieren (1x mit 5000 U/Min./10 Minuten) wurde der Überstand portioniert und bis zur Verwendung bei minus 20°C gelagert. Die Proteinkonzentration betrug bei den beschichteten Platten 4,5µg / Vertiefung.

Beispiel 2: Isolierung und Sequenzierung der für die variablen Bereiche kodierenden cDNA von mAK 6A4

2a) cDNA Isolierung

6A4-Hybridomzellen wurden aus Ascites-Flüssigkeit durch Zentrifugation (10 Min. bei 1500 g und 4°C) abgetrennt. Daraus wurde nach H. Domdey et al., Cell 39, 611-621 (1984) die Poly (A)$^+$ RNA gewonnen. cDNA wurde ausgehend von 15µg Poly (A)$^+$ RNA mit einem kommerziell erhältlichen cDNA-Synthese Kit (Fa. Amersham) synthetisiert. Die doppelsträngige, glattendige cDNA wurde mittels des von Fa. NEN New England Nuclear erhältlichen Klonierungs-Kits mit einzelsträngigen Oligo-dC-Enden versehen und mit entsprechend Oligo-dG-endigem Plasmid pBR322 annelliert. Nach Transformation (Hanahan, D. (1983) J. Mol. Biol. 166, 557-580) von kompetenten E.coli K12 Stamm JM 109 Zellen (Yanisch - Perron C. et al.,(1985) Gene 33, 103-119) wurden etwa 12 000 Transformanten erhalten, die mit $^{32}$P-endmarkiertem Oligonukleotid 5'-CAGGCATCCTAGAGTCAC-3' (I) zum Nachweis von Klonen, die cDNA der schweren Kette von Subgruppe II B enthalten, hybridisiert wurden. Ebenso wurde das mit $^{32}$P-Deoxynukleotidtriphosphaten "nicktranslatierte" 2 850 Basenpaare (bp) große Hind III-Bam HI Fragment (II) des Plasmids C1 (W.O. Weischert et al., (1982) Nucleic Acids Res. 10, 3627-3645) zur Hybridisierung verwendet. (I) stammt aus der Region des IgG2a[a]-Allelsin der Maus (Schwere Kette). (II) kodiert für die Kappa- konstante Region (Leichte Kette). Die Hybridisierungen wurden wie bei D. Woods, Focus 6, 1-2 (1984) beschrieben durchgeführt. Etwa 4.2% bzw. 1.6% der Kolonien reagierten positiv mit den kappa- bzw. gamma-spezifischen Sonden.

2b) cDNA-Sequenzierung

Aus den oben gewonnenen Plasmid-enthaltenden Kolonien wurden solche ausgesucht, die vermutlich komplette cDNA's der leichten bzw. schweren Kette enthielten. Die Insert-DNA der Plasmide pUL5 (enthaltend die cDNA der leichten Kette) und p BH7 (enthaltend die cDNA der schweren Kette) wurden nach Standardmethode sequenziert (F. Sanger et al. (1977) Proc. Natl. Acad. Sci USA 74, 5463-5467), modifiziert wie beschrieben durch E.Y. Chen und P. Seeburg, DNA 4, 165-170 (1985). Die Nukleotidsequenzen der jeweiligen variablen Regionen sind in Tab. 1 (leichte Kette) und Tab. 2 (schwere Kette) angegeben. Für die konstanten Regionen sind die jeweiligen Nukleotidsequenzen - nicht mehr in den Tabellen angegeben - identisch mit den aus der Literatur bekannnten (gamma-Kette: Schreier et al.,- (1981) Proc. Natl. Acad. Sci USA 78, 4495-4499; Kappa-Kette: P.H. Hamlyn et al. (1981) Nucleic Acids Res. 9, 4485-4494). Die Gesamtlänge der kodierenden Regionen beträgt für die leichte Kette 717 bp und für die schwere Kette 1407 bp, wobei die ersten 20 Aminosäuren der leichten Kette das "Leader-Peptid" darstellen, so daß die reife leichte Kette 259 Aminosäuren lang ist. Die schwere Kette besitzt ein "Leader-Peptid" von 19 Aminosäuren, demzufolge das reife Molekül 450 Aminosäuren lang ist.

Tab. 1

EP 0 338 395 B1

```
                                           -60
-96   ctgcagggggggggggggggACAGGCAGTGGGAGCAAGATGGATTCACAGGCCCAGGTTCTT
                                            MetAspSerGlnAlaGlnValLeu
                                            -20

-36   ATATTGCTGCTGCTATGGGTATCTGGTACGTGTGGGGACATTGTGATGTCACAGTCTCCA
-12   IleLeuLeuLeuLeuTrpValSerGlyThrCysGlyAspIleValMetSerGlnSerPro
                                                         1

96    TCCTCCCTGGCTGTGTCAGCAGGAGAGAAGGTCACTATGAGCTGCAAATCCAGTCAGAGT
9     SerSerLeuAlaValSerAlaGlyGluLysValThrMetSerCysLysSerSerGlnSer

156   CTGCTCAACAGTATAACCCGAAAGAACTTCTTGGCTTGGTACCAGCAGAAACCAGGGCAG
29    LeuLeuAsnSerIleThrArgLysAsnPheLeuAlaTrpTyrGlnGlnLysProGlyGln

216   TCTCCTAAACTGCTGATCTACTGGGCATCCACTAGGGAATCTGGGGTCCCTGATCGCTTC
49    SerProLysLeuLeuIleTyrTrpAlaSerThrArgGluSerGlyValProAspArgPhe

276   ACAGGCAGTGGATCTGGGACAGATTTCACTCTCACCATCAGCAGTGTGCAGGCTGAAGAC
69    ThrGlySerGlySerGlyThrAspPheThrLeuThrIleSerSerValGlnAlaGluAsp

336   CTGGCAGTTTATTACTGCAAGCAATCTTATAATCTTCGGACGTTCGGTGGAGGCACCAAG
89    LeuAlaValTyrTyrCysLysGlnSerTyrAsnLeuArgThrPheGlyGlyGlyThrLys

396   CTGGAAATCAAACGGGCTGATGCTGCACCAACTGTATCCATCTTCCCACCATCCAGTGAG
109   LeuGluIleLysArgAlaAspAlaAlaProThrValSerIlePheProProSerSerGlu
                         113
```

Tab. 2

```
-120  ctgcagggggggggAAATACGATCAGCATCCTCTCCACAGACACTGAAAACTCTGACTC

 -60  ACAATGGAAAGGCACTGGATCTTTCTTCCTGTTTCAGTTACTGCAGGTGTCCACTCC
 -19  MetGluArgHisTrpIlePheLeuPheSerValThrAlaGlyValIleSer
      -57   -19

   1  CAGGTCCAGCTTCAGCAGTCTGGGGCTGAACTGGCAAAACCTGGGCCTCAGTGAAGATG
   1  GlnValGlnLeuGlnGlnSerGlyAlaGluLeuAlaLysProGlyAlaSerValLysMet

  61  TCCTGCAAGGCTTCTGGCTACACCTTTACTGCCTACTGGATGCACTGGGTAAAACAGAGG
  21  SerCysLysAlaSerGlyTyrThrPheThrAlaTyrTrpMetHisTrpValLysGlnArg

 121  CCTGGACAGGGTCTGGAATGGATTGGATACATTAATCCTAACACTGGTTATACTGAATAC
  41  ProGlyGlnGlyLeuGluTrpIleGlyTyrIleAsnProAsnThrGlyTyrThrGluTyr

 181  AATCAGAACTTCAAGGACAAGGCCACATTGACTGCAGACAAATCCTCCAGCACAGCCTAC
  61  AsnGlnAsnPheLysAspLysAlaThrLeuThrAlaAspLysSerSerSerThrAlaTyr

 241  ATGCAACTGAGCAGCCTGACATCTGAGGACTCTGAGGCAGTCTATTATTGTACAAGAAGCTAC
  81  MetGlnLeuSerSerLeuThrSerGluAspSerGluAlaValTyrTyrCysThrArgSerTyr

 301  TATAACTACGAGGGGGCTATGGACTACTGGGGTCAAGGAACCTCAGTCACCGTCTCCTCA
 101  TyrAsnTyrGluGlyAlaMetAspTyrTrpGlyGlnGlyThrSerValThrValSerSer

 361  GCCAAAACAACAGCCCCATCGGTCTATCCACTGGCCCCTGTGTGTGGAGATACAACTGGC
 121  AlaLysThrThrAlaProSerValTyrProLeuAlaProValCysGlyAspThrThrGly
```

**Patentansprüche**

**Patentansprüche für folgende Vertragsstaaten : AT, BE, CH, DE, FR, GB, IT, LI, LU, NL, SE**

1. Monoklonaler Antikörper (mAK), dadurch gekennzeichnet, daß die variablen Regionen die Aminosäuresequenzen von Tabelle 1 und Tabelle 2 oder Teile davon enthalten, einschließlich der Allele und Mutanten, soweit diese Teile, Allele und Mutanten Reaktivität mit allen bekannten 19 Serotypen von Pseudomonas aeruginosa besitzen und mit dem äußeren Membranprotein I (OMP I) von Pseudomonas aeruginosa reagieren.

2. Monoklonaler Antikörper nach Anspruch 1, dadurch gekennzeichnet, daß die konstante Region oder Teile davon murinen Ursprungs sind und bevorzugt IgG 2A entsprechen.

**3.** Monoklonaler Antikörper nach Anspruch 1, dadurch gekennzeichnet, daß die konstante Region oder Teile davon humanen Ursprungs sind.

**4.** Hybridom, das einen monoklonalen Antikörper nach Anspruch 1 sezerniert.

**5.** Ein Expressionssystem, das einen monoklonalen Antikörper nach Anspruch 1 exprimiert.

**6.** Epitop, dadurch gekennzeichnet, daß der monoklonale Antikörper nach Anspruch 1 daran bindet.

**7.** Verfahren zur Herstellung eines Hybridoms nach Anspruch 4, dadurch gekennzeichnet, daß ein Säugetier mit den äußeren Membranproteinen von Pseudomonas aeruginosa immunisiert wird, Milzzellen aus einem solchen Tier entnommen, mit Myelomzellen, vorzugsweie NS-1-Zellen fusioniert und die resultierenden Hybridome auf Sezernierung von mAK gegen OMP-I von Pseudomonas aeruginosa selektioniert werden.

**8.** DNA-Sequenzen, kodierend für die in dem monoklonalen Antikörper gemäß Anspruch 1 enthaltenen Aminosäuresequenzen gemäß Tabelle 1 und Tabelle 2 und der gegebenenfalls in diesem Antikörper enthaltenen Teile, Allele und Mutanten dieser Aminosäuresequenzen.

**Patentansprüche für folgende Vertragsstaaten : ES, GR**

**1.** Verfahren zur Herstellung eines monoklonalen Antikörpers, (mAK) dadurch gekennzeichnet, daß die für die variablen Antikörper-Regionen gemäß Tabelle 1 und 2 kodierenden DNA-Sequenzen oder Teile, Allele und Mutanten der variablen Antikörperregionen, soweit diese Teile, Allele und Mutanten Reaktivität mit allen bekannten 19 Serotypen von Pseudomonas aeruginosa besitzen und mit dem äußeren Membranprotein I (OMP I) von Pseudomonas aeruginosa reagieren, in ein geeignetes Antikörper-Gerüst integriert und in geeigneten Expressionssystemen exprimiert werden.

**2.** Verfahren nach Anspruch 1, dadurch gekennzeichnet, daß die konstanten Regionen oder Teile davon murinen Ursprungs sind.

**3.** Verfahren nach Anspruch 2, dadurch gekennzeichnet, daß die konstanten Regionen oder Teile davon humanen Ursprungs sind.

**4.** Verfahren zur Herstellung von Diagnostika, dadurch gekennzeichnet, daß nach Anspruch 1, 2 oder 3 erzeugte mAK eingesetzt werden.

**5.** Verfahren zur Herstellung von Arzneimitteln, dadurch gekennzeichnet, daß pharmazeutische Wirkstoffe an einen nach Anspruch 1, 2 oder 3 hergestelltem mAK gebunden werden.

**6.** Verfahren zur Herstellung von Arzneimitteln, dadurch gekennzeichnet, daß nach Anspruch 1, 2 oder 3 hergestellte mAK mit pharmakologisch üblichen Zusätzen gemischt werden.

**Claims**

**Claims for the following Contracting States : AT, BE, CH, DE, FR, GB, IT, LI, LU, NL, SE**

**1.** A monoclonal antibody (mAb), wherein the variable regions contain the amino acid sequences of Table 1 and Table 2 or parts thereof, including the alleles and mutants, as long as these parts, alleles and mutants possess reactivity with all 19 known serotypes of Pseudomonas aeruginosa and react with outer membrane protein I (OMP I) of Pseudomonas aeruginosa.

**2.** A monoclonal antibody as claimed in claim 1, wherein the constant regions or parts thereof are of murine origin and preferably correspond to IgG2A.

**3.** A monoclonal antibody as claimed in claim 1, wherein the constant regions or parts thereof are of human origin.

**4.** A hybridoma which secretes a monoclonal antibody as claimed in claim 1.

5. An expression system which expresses a monoclonal antibody as claimed in claim 1.

6. An epitope to which a monoclonal antibody as claimed in claim 1 binds.

7. A process for the preparation of a hybridoma as claimed in claim 4, which comprises a mammal being immunized with the outer membrane proteins of Pseudomonas aeruginosa, spleen cells being taken from such an animal and fused with myeloma cells, preferably NS-1 cells, and the resulting hybridomas being selected for secretion of mAbs against OMP-I of Pseudomonas aeruginosa.

8. DNA sequences coding for the amino acid sequences which are shown in Table 1 and Table 2 and are contained in the monoclonal antibody as claimed in claim 1, and for the parts, alleles and mutants, which may be present in said antibody, of these amino acid sequences.

**Claims for the following Contracting States : ES, GR**

1. A process for the preparation of a monoclonal antibody (mAb), which comprises the DNA sequences coding for the variable antibody regions shown in Table 1 and 2, or parts, alleles and mutants of the variable antibody regions, as long as these parts, alleles and mutants possess reactivity with all 19 known serotypes of Pseudomonas aeruginosa and react with outer membrane protein I (OMPI) of Pseudomonas aeruginosa, being integrated in a suitable antibody framework and expressed in suitable expression systems.

2. The process as claimed in claim 1, wherein the constant regions or parts thereof are of murine origin.

3. The process as claimed in claim 2, wherein the constant regions or parts thereof are of human origin.

4. A process for the production of diagnostic aids, which comprises using mAbs produced as claimed in claim 1, 2 or 3.

5. A process for the production of pharmaceuticals, which comprises linking pharmaceutical active substances to an mAb prepared as claimed in claim 1, 2 or 3.

6. A process for the production of pharmaceuticals, which comprises mixing mAbs prepared as claimed in claim 1, 2 or 3 with pharmacologically customary additives.

**Revendications**
**Revendications pour les Etats contractants suivants : AT, BE, CH, DE, FR, GB, IT, LI, LU, NL, SE**

1. Anticorps monoclonal (AcM), caractérisé en ce que les régions variables contiennent les séquences d'aminoacides du tableau 1 et du tableau 2 ou des parties de celles-ci, y compris des allèles et mutants, dans la mesure où ces parties, allèles et mutants possèdent une réactivité avec tous les 19 sérotypes connus de *Pseudomonas aeruginosa* et réagissent avec la protéine de membrane externe I (OMP I) de *Pseudomonas aeruginosa*.

2. Anticorps monoclonal selon la revendication 1, caractérisé en ce que la région constante ou des parties de celle-ci sont d'origine murine et correspondent de préférence à IgG 2A.

3. Anticorps monoclonal selon la revendication 1, caractérisé en ce que la région constante ou les parties de celle-ci sont d'origine humaine.

4. Hybridome sécrétant un anticorps monoclonal selon la revendication 1.

5. Système d'expression exprimant un anticorps monoclonal selon la revendication 1.

6. Epitope, caractérisé en ce que l'anticorps monoclonal selon la revendication 1 se lie à celui-ci.

7. Procédé pour la production d'un hybridome selon la revendication 4, caractérisé en ce que l'on immunise un mammifère avec les protéines de membrane externes de *Pseudomonas aeruginosa*, on prélève des cellules spléniques d'un tel animal, on les fait fusionner avec des cellules de myélome, de préférence des cellules NS-1, et on sélectionne les hybridomes résultants sur la base de la sécrétion d'AcM dirigés contre OMP-I de *Pseudomonas aeruginosa*.

8. Séquences d'ADN codant pour les séquences d'aminoacides selon le tableau 1 et le tableau 2, contenues dans l'anticorps monoclonal selon la revendication 1, et les parties, allèles et mutants de ces séquences d'aminoacides, éventuellement contenus dans cet anticorps.

**Revendications pour les Etats contractants suivants : ES, GR**

1. Procédé pour la production d'un anticorps monoclonal (AcM), caractérisé en ce que les séquences d'ADN codant pour les régions variables de l'anticorps selon les tableaux 1 et 2 ou des parties, allèles et mutants des régions variables de l'anticorps, dans la mesure où ces parties, allèles et mutants possèdent une réactivité avec tous les 19 sérotypes connus de *Pseudomonas aeruginosa* et réagissent avec la protéine de membrane externe I (OMP I) de *Pseudomonas aeruginosa*, sont intégrées dans un squelette d'anticorps approprié et exprimées dans des systèmes d'expression appropriés.

2. Procédé selon la revendication 1, caractérisé en ce que les régions constantes ou les parties de celles-ci sont d'origine murine.

3. Procédé selon la revendication 2, caractérisé en ce que les régions constantes ou les parties de celle-ci sont d'origine humaine.

4. Procédé pour la production d'agents de diagnostic, caractérisé en ce que l'on utilise des AcM produits selon la revendication 1, 2 ou 3.

5. Procédé pour la fabrication de médicaments, caractérisé en ce que des substances actives pharmaceutiques sont liées à un AcM produit selon la revendication 1, 2 ou 3.

6. Procédé pour la fabrication de médicaments, caractérisé en ce que l'on mélange des AcM, produits selon la revendication 1, 2 ou 3, avec des additifs pharmacologiquement usuels.